# EUROPEAN PATENT SPECIFICATION

(11) **EP 1 942 090 B1**
(45) Date of publication and mention of the grant of the patent: **23.10.2013**
(21) Application number: 08005540.3
(22) Date of filing: 24.01.2002
(51) Int. Cl.: C07C 11/04, C07C 11/107, C07C 6/04, C07C 5/25

(54) **Process for the production of hexene-1 and ethylene**
Verfahren zur Herstellung von 1-Hexen und Ethylen
Procédé pour la production d'hexène-1 et d'éthylène

(30) Priority: 25.01.2001 US 263924 P
(43) Date of publication of application: 09.07.2008
(62) Divisional of application: 02703253.1
(73) Proprietor: Lummus Technology Inc., Bloomfield, NJ 07003 (US)
(72) Inventor: Gartside, Robert, J., Summit, NJ 07901 (US)
(74) Representative: Giovannini, Francesca

(56) References cited:
- EP-A- 1 134 271
- GB-A- 1 471 151
- US-A- 3 776 974
- US-A- 4 368 345
- US-A- 5 057 638

## Description

This invention relates to the production of linear alpha olefins. More particularly, this invention relates to the production of linear alpha olefins having a higher carbon number from alpha olefins having a lower carbon number. More particularly, this invention relates to the production of ethylene and hexene-1.

US 5,057,638 describes a process for making 1-hexene comprising:
A. metathesizing 1-butene to a mixture comprising 3-hexene and ethylene;
B. separating the 3-hexene from the product of step A;
C. reacting the 3-hexene with an electrophilic compound containing reactive hydrogen under acidic catalyzed conditions which permit the electrophilic compound containing reactive hydrogen to add to carbon-carbon double bonds; and
D. cracking the product ofstep C to produce a mixture of n-hexenes containing 1-hexene.

Linear alpha olefins currently are produced by the selective oligomerization of.ethylene.

In general, the oligomerization is conducted in the presence of a catalyst, such as an alkylated metal catalyst. Long residence times are used to produce hydrocarbon chains of varying lengths. The olefin double bond remains at the alpha position as each ethylene molecule is added. Ethylene oligomerization produces a wide spectrum of linear alpha olefin products. Extensive fractionation is required to separate the alpha olefins having different carbon numbers.

According to a first aspect, the present invention relates to a process for converting butene-1 to ethylene and hexene-1, said process comprising: (a) subjecting a feed comprising butene-1 to catalytic metathesis under conditions and with a metathesis catalyst that minimises isomerisation of said feed to produce ethylene, propylene, pentene-2 and hexene-3, wherein such catalyst and conditions are selected in order to achieve a weight selectivity to hexene-3 of at least 40 % from butene-1; wherein the portion of the feed that contains C₄ olefins is at least 90% butene-1. and wherein metathesis is effected at a WHSV of from 3 to 200, at a pressure of from 0.170 MPa (10 psig) to 4.24 MPa (600 psig), and at a temperature of from 50°C to 600°C; (b) recycling the pentene-2 to the metathesis reaction; and (c) isomerization of hexene-3 to hexene-1.

In accordance with the present invention, a linear alpha-olefin having a first number of carbon atoms is subjected to a metathesis reaction under conditions and in the presence of a catalyst that minimizes or eliminates, skeletal and/or double bond isomerization to produce a reaction product that includes ethylene and a linear internal olefin (LIO) that has a number of carbon atoms greater than the first number of carbon atoms. Specifically, the feed linear alpha olefin with carbon number is metathesized to ethylene and linear internal olefin with carbon number 2n-2. The resulting linear internal olefin may then be isomerized to produce a linear alpha olefin.

In accordance with the present invention, butene-1 is subjected to a metathesis reaction under conditions and in the presence of a metathesis catalyst that minimizes or eliminates skeletal and double bond isomerization to produce a reaction product that includes ethylene and hexene-3. The hexene-3 is then isomerized to hexene-1.

In the metathesis reaction, the catalyst and reaction conditions are such as to minimize isomerization of the butene-1 starting material.

The catalyst used in this aspect for the metathesis reaction may be a supported or unsupported catalyst and the overall catalyst is one that has a minimized amount of both acidic and basic sites.

A catalyst that has a reduced amount of both acidic and basic sites (preferably essentially no acidic and basic sites) improves the selectivity of the metathesis of the lower carbon number alpha-olefin ( 1-butene) with itself to form ethylene and a linear internal olefin of higher carbon number ( 3- hexene) by minimizing isomerization.

Metathesis reactions can be characterized as either fully productive, half productive, or non-productive depending upon the symmetry of the molecule with respect to the double bond. Two dissimilar olefins will react in a "fully productive" manner. An example is the reaction of 1-butene with 2-butene. The double bond is in a different position in the respective molecule and these will react rapidly with one another. Two identical molecules will react in either a half productive or non-productive manner. If for example 1-butene reacts with itself, because the double bond is in the same position within each molecule, it will react at a rate considerably slower than the rate of fully productive reactions. If the feed molecules are identical and symmetric around the double bond (for example 2-butene reacting with itself), then no reaction will occur and the system is defined as non-productive.

In many metathesis reaction systems, isomerization activity is incorporated to increase the rates of reaction. By allowing for a shifting double bond, half or non-productive reactions can be reduced. Typical isomerization catalysts include basic metal oxides or promoted zeolites.

In one preferred embodiment, the catalyst or catalyst mixture contains essentially no magnesium oxide in that magnesium oxide catalyst promotes isomerization. Thus, for example, a preferred catalyst (supported or not supported) is a Group VI B or Group VII B metal oxide such as tungsten oxide, molybdenum oxide, or rhenium oxide, with tungsten oxide being particularly preferred.

If a support is used, such support has a minimized amount of both acidic and basic sites and preferably essentially no acid and essentially no basic sites. Preferred supports are silica or zirconia oxide.

The catalyst may include a promoter to reduce acidity; for example, an alkali metal (sodium, potassium or lithium), cesium, a rare earth, etc.

Generally reaction conditions that tend to favor the primary reaction and discourage subsequent reactions are preferred. Thus a lower pressure and shorter residence times tend to minimize the isomerization reaction.

In accordance with the present invention, linear alpha olefin butene-1 is subjected to auto-metathesis with itself (half productive reaction) in the presence of a catalyst and under conditions that minimize isomerization of the Butene-1 to produce a reaction product that includes ethylene and a linear alpha olefin of carbon number 2n-2, such as hexene-3. The reaction is:

1-C₄⁻ + 1-C₄⁻ ⇄ 3-1-C₆⁻

The metathesis reaction, in accordance with the invention is effected at a temperature from about 50°C to 600°C, preferably from about 200°C to 350°C, at a weight hourly space velocity (WHSV) of from about 3 to about 200, preferably from about 6 to about 40, and at a pressure of from about 0.170 MPa (10 psig) to about 4.24 MPa (600 psig), preferably from about 0.308 MPa (30 psig) to about 0.791 MPa (100 psig).

The butene-1 starting material may be a pure or impure feed,. The portion of the feed that contains reactive C₄ olefins is at least 90% and more preferably at least 95% butene-1. Non reactive components , for example normal butane, may dilute the olefins in the feedstock. Most preferably, the butene-1 starting material is a pure feed i.e. the butene-1 is present in an amount of at least 99%.

In one embodiment, a mixed butene feed is pretreated to provide a butene-1 feed for the metathesis reaction. For example, the mixed butene feed may contain butene-1, butene-2 and isobutylene. Such a feed may be obtained from a steam cracker In the event that the mixed butene stream includes butadiene such butadiene is removed by hydrogenation or extraction.

In one embodiment, the mixed butenes feed following or in conjunction with butadiene hydrogenation is subjected to hydroisomerization conditions to convert butene-1 to butene-2, with isobutylene being separated from a butene-2 stream by fractionation. The butene-2 stream is then isomerized back to butene-1 in a subsequent step for use as feed to the metathesis portion of the process

The hydro isomerization of butene-1 to butene-2 and separation may be accomplished in separate steps or may be combined into a single step. In one embodiment butene-1 is isomerized to' butene-2 and isobutylene is separated from butene-2 , in a catalytic distillation tower In another embodiment; the isomerization of the butene-1 to butene-2 can also be accomplished by using a fixed bed isomerization reactor or by adjusting the conditions of the butadiene hydrogenation reactor followed by a fractionation tower to produce a butene-2 stream and an isobutylene product.

The isomerization of 1-butene to 2-butene is favored by low temperatures. This reaction is carried out typically in liquid phase either in a catalytic distillation tower or fixed bed reactor as described above.

The subsequent isomerization of 2-butene to 1-butene is favored by higher temperature and preferably is carried out in a vapor fixed bed phase reactor. Alternately, the conversion of butene-2 to butene-1 and the purification of the butene-1 may be accomplished by catalytic distillation. The production of 1-butene from hydrocarbons fed to a steam cracker is described further in U.S. Patent No. 5,087,780.

As a further alternative, a mixed butene stream is reacted with for example methanol to convert isobutylene to methyl tertiary butyl ether (MTBE) as known in the art. The MTBE product is removed from the butenes stream. The resultant mixed normal butenes stream (butene-1 and butene-2) is then processed in a similar manner to the butene-2 stream above to produce an essentially pure butene-1 stream for feed to the metathesis reactor.

Thus, in accordance with an embodiment of the present invention a mixed C4 stream is converted to ethylene and hexene-1 by the steps of a. hydrogenating the butadiene to 1 and 2 butenes, b. converting butene-1 to butene-2 and separating isobutylene therefrom, c. isomerization of butene-2 to produce butene-1, d. separation of butene 1 from butene 2 to produce an essentially pure butene 1 stream, e. autometathesis of butene-1under non-isomerizing conditions to produce ethylene and hexene-3, and f. isomerizing hexene-3 to hexene-1.

The effluent from the metathesis reactor is passed to a series of fractionation towers, where ethylene is removed, unconverted butenes are separated for recycle, and the hexene fraction is removed from the bottoms. The bottoms stream, which is primarily 3-hexene, is isomerized to 1-hexene and the 1-hexene purified by fractionation.. In a preferred embodiment, isomers of 2-hexene and 3 hexene are reacted essentially to extinction by recycle. The isomerization reaction schemes for hexene are essentially the same as described above for conversion of 2-butene to 1-butene. The overhead product is 1-hexene.

The hexene-3 produced in the autometathesis is isomerized in the presence of a suitable catalyst. The reaction can take place either in the liquid phase or the vapor phase. For liquid phase reaction, such catalysts include, but are not limited to, palladium and platinum catalysts. The catalyst may be supported on a suitable support material, such as alumina, for example. The reaction occurs in the presence of small amounts of hydrogen (hydroisomerization).

For vapor phase reaction, such catalysts include, but are not limited to, basic metal oxides, including magnesium oxide. In accordance with the invention, the metathesis reaction of the butene-1 occurs first, followed by the isomerization of the hexene-3 product from that reaction. In this case, no hydrogen is required. The equilibrium of mixed hexenes to 1-hexene is favored by higher temperatures hence the vapor phase reaction occurring at higher temperatures is preferred.

The hexene-3 may be isomerized to hexene-1 at a WHSV of from about 3 to about 200, preferably from about 10 to about 60, and at a pressure of from about 2 bar to about 40 bar, preferably from about 3 bar to about 10 bar, and a temperature from about 40°C to 300°C, preferably from about 60°C to 150°C. for liquid phase and 300 to 400°C for vapor phase reaction.

Although in accordance with the present invention, the metathesis reaction is effected with a catalyst and under conditions that minimize isomerization, some isomerization occurs. As a result of the isomerization, the internal olefin butene-2, is produced which reacts with the feed linear alpha olefin butene-1, to produce propylene and a non-selective linear internal olefin pentene-2, via the reaction:

1-C4- + 2-C4- ⇄ C3-+2-C5-

The propylene may be recovered as reaction product; however, in many cases, the pentene-2 is less valuable. In accordance with the invention, pentene-2 is recycled to the metathesis reaction. The pentene-2 reacts with butene-1 to produce propylene and hexene-3 via the reaction:

1-C4- +2-C5 → C3-+3-C6-

For example, in the metathesis of butene-1 under minimum isomerization conditions, there is about a 35 mol % selectivity of each of ethylene and hexene and 15 mol % of each of propylene and 2-pentene. This gives a 20% wt. selectivity to 2-C₅H₁₀. If the metathesis feed in addition to the 1-butene includes a stream of 2-pentene, such that the 2-pentene concentration in the feed is 10%, the net pentene selectivity goes to essentially 0 and the hexene selectivity increases from 35 to 46% molar or over 60% by weight. In this manner, hexene selectivity is increased.

In addition to participating in the above reaction, the presence of 2-pentene suppresses the formation of additional 2C5 by limiting the non-selective reaction of 1-butene with 2-butene due to equilibrium and ultimately limiting the isomerization of 1-butene to 2-butene because 2-butene formed is not reacting away, thus creating an additional equilibrium limitation.

In accordance with the present invention, the metathesis reaction of 1-butene is effected at conditions and with a catalyst that minimizes isomerization in order to increase selectivity to hexene-3 and ethylene. In particular, such catalyst and conditions are selected in order to achieve a weight selectivity to hexene-3 of at least 40% and preferably at least 50% or greater from 1-butene (without recycle) and still higher values when incorporating pentene recyle.

The invention now will be described with respect to the following example.

### EXAMPLE 1

In this example, a catalyst consisting of WO₃ on a silica support is loaded into a reactor either alone, or admixed with MgO prior to loading. Pure 1- butene is passed over the catalyst at a WHSV of 13 and at various pressures as shown in Table 1 below. The reaction temperature is 650°F (343°C) over the catalyst. Selectivity is calculated by dividing the weight of each product by the weight loss of 1-butene converted to products. The selectivities to various components are given in Table 1 below.

**Table 1**

| **Weight percent Selectivity of 1-butene conversion to products** | | | | | | | | |
|---|---|---|---|---|---|---|---|---|
| | 3/1 MgO/WO3 | | 1/1 MgO/WO3 | | | Pure WO3 | Pure WO3 | Pure WO3 |
| Residence Time | | | | | | | | |
| | 8 sec | | 12 sec | | | 12 sec | 4 sec | 2 sec |
| | 1.896 MPa | 1.896 MPa | 3.103 MPa | 3.103 MPa | | 3.103 MPa | 1.034 MPa | 0.517 MPa |
| Pressure | (275 psi) | (275 psi) | (450 psi) | (450 psi) | | (450 psi) | (150 psi) | (75 psi) |
| C2- | 2.5 | 2.5 | 2.1 | 2.4 | | 13.9 | 17.8 | 17.4 |
| C3- | 26.3 | 22.9 | 23.3 | 23.9 | | 14.9 | 9.2 | 9.1 |
| 2-C4- | 31.2 | 31.6 | 32.8 | 30.4 | | 2.8 | 1.0 | 0.6 |
| 2-C5- | 19.2 | 18.1 | 24.8 | 25.0 | | 16.9 | 11.0 | 11.2 |
| 1-C5- | 2.5 | 2.5 | 1.8 | 2.0 | | 5.8 | 3.6 | 1.8 |
| C6 | 8.4 | 12.1 | 10.1 | 10.8 | | 36.2 | 50.5 | 54.1 |
| C7 | 2.3 | 5.5 | 2.1 | 2.3 | | 4.8 | 3.1 | 1.9 |
| C8+ | 3.7 | 5.5 | 1.0 | 1.0 | | 2.0 | 1.3 | 3.9 |

As shown in Table 1 above, both the 3/1 and the 1/1 ratio mixtures of MgO and WO₃-SiO₂ catalyst show low weight selectivity to ethylene and C₆ alkenes. The isomerization activity of the MgO effectively converts some portion of the 1 butene to 2-butene and the metathesis reaction of:

1-C₄H₈ + 2-C₄H₈ → C₃H₆ + 2-C₅H₁₀

occurs limiting.the auto-metathesis reaction of 1-C₄ H₈ to.form C₂H₄ + 3-C₆H₁₂.

When a pure WO₃-SiO₂ catalyst is employed, the selectivity for hexene increased from 8.4% - 12.1% to as high as 54.1% hexene. The theoretical weight selectivities for the pure autometathesis reaction itself (no isomerization) are 25 wt. % ethylene and 75 wt. % hexene. Thus, reducing the isomerization activity of the catalyst system improved selectivity to hexene.

### EXAMPLE 2

In this example, a feed containing pure 1-butene, and feeds containing 1-butene mixed with 2-butene or isobutylene are reacted in a reactor in the presence of a catalyst consisting of WO₃ and silica support at a temperature of 315.56°C (600°F) or 343.33°C (650°F), and a pressure of 0.618 MPa (75 psig). These condition and catalyst are preferred to minimize isomerization reactions. Selectivity is calculated in wt. % or mol. % by dividing the weight and number of moles of each product by the weight loss and molar loss of 1-butene converted to products. The selectivities to various components are given in Table 2 below.

**Table 2**

| | **Feed (wt.** %**)** | | |
|---|---|---|---|
| | **A** | **B** | **C** |
| 1-C₄ | 100 | 75 | 95 |
| 2-C₄ | 0 | 25 | 0 |
| i-C₄ | 0 | 0 | 5 |
| Temp. (°C) | (650°F) 343.33 | (600°F) 315.56 | (600°F) 315.56 |
| Pressure (MPa) | (75psig) 0.618 | (75psig) 0.618 | (75 psig) 0.618 |

| Conversion (%) | | | |
|---|---|---|---|
| 1-C₄ | 47 | 52 | 56 |
| 2-C₄ | N/A | 66 | N/A |
| i-C₄ | N/A | N/A | 31 |

| Selectivity (mol %) | | | |
|---|---|---|---|
| C₂H₄ | 37 | 15 | 31 |
| 3-C₆H₁₂ | 38 | 19 | 28 |
| C₃H₆ | 12 | 34 | 19 |
| 2-C₅H₁₀ | 11 | 29 | 18 |
| Others | 2 | 3 | 4 |

| Selectivity (wt.%) | | | |
|---|---|---|---|
| C₂H₄ | 18.5 | 7.5 | 15.5 |
| 3-C₆H₁₂ | 57 | 28.5 | 42 |
| | | | |
| C₃H₆ | 9 | 25.5 | 14 |
| 2-C₅H₁₀ | 14 | 36.0 | 22.5 |
| Others | 1.5 | 2.5 | 6 |

The above results show that when the feed containing 1-butene is a feed of pure 1-butene with no other components, there is increased selectivity to 3-hexene, as compared with feeds containing 2-butene or isobutylene along with 1-butene.

### EXAMPLE 3

In this example, a feed containing essentially pure 1-butene (95 % 1-butene and 5 % iso-butene) and a feed containing 1-butene, iso-butene and 10 % 2-pentene are reacted in a reactor in the presence of a catalyst consisting of WO₃ and a silica support at a temperature of 343.33°C (650°F), and a pressure of 0.618 MPa (75 psig). These conditions and catalyst are preferred to minimize isomerization reactions. Selectivity is calculated in wt. % and mol. % by dividing the weight and number of moles of each product by the weight loss and molar loss of 1-butene converted to products. The selectivities to various components are given in Table 3 below.

**Table 3**

| | **Feed (wt. %)** | |
|---|---|---|
| | **A** | **B** |
| 1-C₄ | 95 | 87 |
| 1-C₄- | 5 | 3 |
| 2-C₅ | 0 | 10 |
| Temp. (°C) | (650°F) 343.33 | (650°F) 343.33 |
| Pressure (MPa) | (75 psig) 0.618 | (75 psig) 0.618 |

| Conversion (%) | | |
|---|---|---|
| 1-C₄ | 56 | 47 |
| 2-C₅ | N/A | 26 |
| i-C₄ | 31 | 29 |

| Selectivity (mol %) | | |
|---|---|---|
| C₂H₄ | 31 | 34 |
| 3-C₆H₁₂ | 28 | 43 |
| C₃H₆ | 19 | 17 |
| 2-C₅H₁₀ | 18 | 1.6 |
| Others | 4 | 4.4 |

| Selectivity (wt.%) | | |
|---|---|---|
| C₂H₄ | 15.5 | 16.9 |
| 3-C₆H₁₂ | 42 | 64.1 |
| | | |
| C₃H₆ | 14 | 12.7 |
| 2-C₅H₁₀ | 22.5 | 2.0 |
| Others | 6 | 4.3 |

The above results show that when the 2-pentene produced by the non-selective isomerization of the low isomerization catalyst is recycled, there is increased selectivity to 3-hexene, as compared with feed where the 2-pentene is not recycled to the reactor..If the base feedstock was a pure 1-butene stream, the increase in hexene selectivity would be even greater.

### EXAMPLE 4

In this example, feeds containing essentially pure 1-butene (99.9% 1- butene), and feeds containing 1-butene and 2-pentene are reacted in a reactor in the presence of Catalyst A or Catalyst B at a temperature of 343.33°C (650°F) and a pressure of 0.618 MPa (75 psig). These conditions are preferred to minimize isomerization reactions. Catalyst A consists of WO₃ and a silica support, and Catalyst B consists of WO₃ and a special chromatographic grade silica support. Catalyst A uses a silica support that contains 2,000 ppm sulfur, thus creating acidic reaction sites. Catalyst B uses a silica support where the sulfur has been reduced to less than 100 ppm. Both catalysts have a low isomerization activity, and the isomerization activity of Catalyst B is lower than that of Catalyst A. Selectivity is calculated in wt. % and mol% by dividing the weight and number of moles of each product by the weight loss and molar loss of 1-butene converted to products. The selectivities to various components are given in Table 4 below.

**Table 4**

| **Feed (wt. %)** | | | | |
|---|---|---|---|---|
| Catalyst | **A** | **A** | **B** | **B** |
| 1-C₄ | 99.9 | 87.6 | 99.9 | 91.3 |
| 2-C₅ | 0 | 10.2 | 0 | 8.7 |
| Temp. (°C) | (650 °F) 343.33 | (650 °F) 343.33 | (650 °F) 343.33 | (650 °F) 343.33 |
| Pressure (MPa) | (75 psig) 0.618 | (75 psig) 0.618 | (75 psig) 0.618 | (75 psig) 0.618 |

| Conversion (%) | | | | |
|---|---|---|---|---|
| 1-C₄ | 56 | 45.4 | 41 | 38 |
| 2-C₅ | Net Production | 26.6 | Net Production | 32.5 |
| i-C₄ | N/A | 24.4 | N/A | N/A |

| Selectivity (mol%) | | | | |
|---|---|---|---|---|
| C₂H₄ | 31 | 35.5 | 44.2 | 38.4 |
| 3-C₆H₁₂ | 28 | 46.2 | 45.8 | 47.8 |
| C₃H₆ | 19 | 15.5 | 5.2 | 11.6 |
| 2-C₅H₁₀ | 18 | Net Conversion | 4.4 | Net Conversion |
| Others | 4 | 2.8 | 0.4 | 2.2 |

| Selectivity (wt. %) | | | | |
|---|---|---|---|---|
| C₂H₄ | 15.5 | 17.8 | 22.1 | 19.2 |
| 3-C₆H₁₂ | 42 | 69.3 | 68.6 | 71.7 |
| | | | | |
| C₃H₆ | 14 | 11.6 | 3.8 | 8.7 |
| 2-C₅H₁₀ | 22.5 | Net Conversion | 5.3 | Net Conversion |
| Others | 6 | 1.3 | 0.2 | 0.4 |

As can be seen in Table 4 above, the recycling of pentenes increases hexene selectivity and provides for a reduced pentene make. In these tests, between 26 and 38% of the pentenes in the feed were converted when approximately 10% pentene was present in the feed. The selectivity increase is most significant when greater amounts of pentenes are produced. Note further, however, when using catalyst systems with high isomerization activity, the amounts of pentenes formed are so large as to render recycle impractical and very costly. This effect is most significant when using low isomerization activity catalysts.

## Claims

1. A process for converting butene-1 to ethylene and hexene-1, said process comprising:
(a) subjecting a feed comprising butene-1 to catalytic metathesis under conditions and with a metathesis catalyst that minimises isomerisation of said feed to produce ethylene, propylene, pentene-2 and hexene-3, wherein such catalyst and conditions are selected in order to achieve a weight selectivity to hexene-3 of at least 40 % from butene-1;
wherein the portion of the feed that contains C₄ olefins is at least 90% butene-1; and
wherein metathesis is effected at a WHSV of from 3 to 200, at a pressure of from 0.170 MPa (10 psig) to 4.24 MPa (600 psig), and at a temperature of from 50°C to 600°C;
(b) recycling the pentene-2 to the metathesis reaction; and
(c) isomerization of hexene-3 to hexene-1.

2. The process of claim 1, wherein said weight selectivity to hexene-3 from butene-1 is at least 50 %.

3. The process of claim 1, wherein said catalyst is selected from the group consisting of tungsten oxide, molybdenium oxide, rhenium oxide and mixtures thereof.

4. The process of claim 3, wherein said catalyst is tungsten oxide.

5. The process of claim 4, wherein said catalyst is supported tungsten oxide.

6. The process of claim 1, wherein said metathesis is effected at a WHSV of from 6 to 40.

7. The process of claim 1, wherein said metathesis is effected at a pressure of from 0.308 MPa (30 psig) to 0.791 MPa (100 psig).

8. The process of claim 4, wherein said catalyst is supported on a silica support.

9. The process of claim 1, wherein said hexene-3 is isomerised to hexene-1 at WHSV of from 3 to 200, at a pressure of from 0.2 MPa (2 bar) to 4 MPa (40 bar), and at a temperature of from 40°C to 300°C for liquid phase and at a temperature of from 300°C to 400°C for vapor phase reaction.

10. The process of claim 9, wherein said hexene-3 is isomerised to hexene-1 at a pressure of from 0.3 MPa (3 bar) to 1 MPa (10 bar).

11. The process of claim 9, wherein said hexene-3 is isomerised to hexene-1 at a temperature of from 60°C to 150°C for liquid phase.

## Patentansprüche

1. Verfahren zur Umwandlung von Buten-1 in Ethylen und Hexen-1, wobei das Verfahren umfasst:
(a) katalytische Metathese an einem Buten-1 umfassenden Zustrom unter Bedingungen und mit einem Metathesekatalysator, der die Isomerisierung des Zustroms auf ein Mindestmaß beschränkt, zur Bildung von Ethylen, Propylen, Penten-2 und Hexen-3, wobei der Katalysator und die Bedingungen geeignet ausgewählt werden, um eine Gewichtsselektivität für Hexen-3 aus Buten-1 von mindestens 40 % zu erhalten,
wobei es sich bei dem Anteil des Zustroms, der C₄-Olefine enthält, um mindestens 90 % Buten-1 handelt, und
wobei die Metathese bei einer Raumgeschwindigkeit WHSV im Bereich von 3 bis 200, bei einem Druck im Bereich von 0,170 MPa (10 psig) bis 4,24 MPa (600 psig) und bei einer Temperatur im Bereich von 50°C bis 600°C durchgeführt wird,
(b) Rückführung des Penten-2 zu der Metathesereaktion und
(c) Isomerisierung von Hexen-3 zu Hexen-1.

2. Verfahren nach Anspruch 1, wobei die Gewichtsselektivität für Hexen-3 aus Buten-1 mindestens 50% beträgt.

3. Verfahren nach Anspruch 1, wobei der Katalysator aus der Gruppe ausgewählt ist, die aus Wolframoxid, Molybdänoxid, Rheniumoxid und Mischungen derselben besteht.

4. Verfahren nach Anspruch 3, wobei es sich bei dem Katalysator um Wolframoxid handelt.

5. Verfahren nach Anspruch 4, wobei es sich bei dem Katalysator um geträgertes Wolframoxid handelt.

6. Verfahren nach Anspruch 1, wobei die Metathese bei einer Raumgeschwindigkeit WHSV im Bereich von 6 bis 40 durchgeführt wird.

7. Verfahren nach Anspruch 1, wobei die Metathese bei einem Druck im Bereich von 0,308 MPa (30 psig) bis 0,791 MPa (100 psig) durchgeführt wird.

8. Verfahren nach Anspruch 4, wobei der Katalysator auf einem Siliziumdioxidträger geträgert ist.

9. Verfahren nach Anspruch 1, wobei das Hexen-3 bei einer Raumgeschwindigkeit WHSV im Bereich von 3 bis 200, bei einem Druck im Bereich von 0,2 MPa (2 bar) bis 4 MPa (40 bar) und einer Temperatur im Bereich von 40°C bis 300°C für die Flüssigphasen- und bei einer Temperatur im Bereich von 300°C bis 400°C für die Dampfphasenreaktion zu Hexen-1 isomerisiert wird.

10. Verfahren nach Anspruch 9, wobei das Hexen-3 bei einem Druck im Bereich von 0,3 MPa (3 bar) bis 1 MPa (10 bar) zu Hexen-1 isomerisiert wird.

11. Verfahren nach Anspruch 9, wobei das Hexen-3 bei einer Temperatur im Bereich von 60°C bis 150°C für die Flüssigphase zu Hexen-1 isomerisiert wird.

## Revendications

1. Procédé de conversion de butène-1 en éthylène et hexène-1, ledit procédé comprenant de :
(a) soumettre une alimentation comprenant du butène-1 à une métathèse catalytique dans des conditions et avec un catalyseur de métathèse qui minimise l'isomérisation de ladite alimentation pour produire de l'éthylène, du propylène, du pentène-2 et de l'hexène-3, le catalyseur et les conditions étant sélectionnés afin d'obtenir une sélectivité en poids de l'hexène-3 d'au moins 40 % du butène-1 ;
dans lequel la partie de l'alimentation qui contient des oléfines en C₄ se compose d'au moins 90 % de butène-1 ; et
dans lequel la métathèse est réalisée à une vitesse spatiale horaire en poids (WHSV) de 3 à 200, à une pression de 0,170 MPa (10 psig) à 4,24 MPa (600 psig), et à une température de 50 °C à 600 °C ;
(b) recycler le pentène-2 vers la réaction de métathèse ; et
(c) isomériser l'hexène-3 en hexène-1.

2. Procédé selon la revendication 1, dans lequel ladite sélectivité en poids de l'hexène-3 par rapport au butène-1 est d'au moins 50 %.

3. Procédé selon la revendication 1, dans lequel ledit catalyseur est sélectionné dans le groupe consistant en un oxyde de tungstène, un oxyde de molybdène, un oxyde de rhénium et des mélanges de ceux-ci.

4. Procédé selon la revendication 3, dans lequel ledit catalyseur est un oxyde de tungstène.

5. Procédé selon la revendication 4, dans lequel ledit catalyseur est un oxyde de tungstène supporté.

6. Procédé selon la revendication 1, dans lequel ladite métathèse est réalisée à une WHSV de 6 à 40.

7. Procédé selon la revendication 1, dans lequel ladite métathèse est réalisée à une pression de 0,308 MPa (30 psig) à 0,791 MPa (100 psig).

8. Procédé selon la revendication 4, dans lequel ledit catalyseur est supporté sur un support de silice.

9. Procédé selon la revendication 1, dans lequel ledit hexène-3 est isomérisé en hexène-1 à une WHSV de 3 à 200, à une pression de 0,2 MPa (2 bar) à 4 MPa (40 bar) et à une température de 40 °C à 300 °C pour une réaction en phase liquide et à une température de 300 °C à 400 °C pour une réaction en phase vapeur.

10. Procédé selon la revendication 9, dans lequel ledit hexène-3 est isomérisé en hexène-1 à une pression de 0,3 MPa (3 bar) à 1 MPa (10 bar).

11. Procédé selon la revendication 9, dans lequel ledit hexène-3 est isomérisé en hexène-1 à une température de 60 °C à 150 °C pour une réaction en phase liquide.
